(19) Europäisches Patentamt
European Patent Office
Office européen des brevets

(11) **EP 3 671 729 A1**

(12) **EUROPEAN PATENT APPLICATION**

(43) Date of publication:
**24.06.2020 Bulletin 2020/26**

(51) Int Cl.:
***G10K 11/175*** (2006.01)          ***A61B 5/0476*** (2006.01)
***A61B 5/00*** (2006.01)            ***A61M 21/00*** (2006.01)

(21) Application number: **18212981.7**

(22) Date of filing: **17.12.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **VAN DEN ENDE, Daan Anton
5656 AE Eindhoven (NL)**
• **PASTOOR, Sander Theodoor
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **A NOISE MASKING DEVICE AND A METHOD FOR MASKING NOISE**

(57) The invention provides a device (and method) for masking noise in which a calibration is carried out to determine the sensitivity of a user to a calibration sound. During use of the device, the signal characteristics of the masking sound are adjusted based on the detected noise, the response of the user to the detected noise and also the response of the user to the calibration sound. As a result, a masking sound is generated that is optimally adapted to mask unwanted noise, in particular in a way which avoids the masking noise itself becoming a disturbance to the particular user.

FIG. 1

**Description**

FIELD OF THE INVENTION

The present invention relates to the field of systems for mitigating unwanted acoustic noise, and in particular to the field of noise masking systems.

BACKGROUND OF THE INVENTION

Unwanted acoustic noise can disturb people or subjects. The disturbance to a person can be mitigated by playing a masking or anti-noise (i.e. noise-cancelling) sound. Such sounds can be generated and played through an external device (e.g. speakers or a smartphone), or can be generated and played by hardware forming part of a noise masking system.

A masking sound is typically a recorded repetitive sound (such as rain or ocean waves) or a generated random waveform with equally distributed acoustic intensity over the audible frequency range (termed 'white noise'). These sounds all aim to drown out sudden and/or annoying external noise and can be clustered under the term 'masking sound'.

In particular, a masking sound can mask acoustic noise that would otherwise disturb a user, e.g. during sleep.

Anti-noise (sound cancellation) is a special form of masking sound which needs a microphone close to the ear to pick up the sound vibrations in order to play the right phase-shifted anti-noise.

A masking sound volume should be high enough to drown out the unwanted noise. However, noise levels may change, which would necessitate a corresponding different volume for the masking sound. Typical masking sound generators operate without feedback. Thus, the user must manually balance the volume settings to drown out the unwanted noise while also avoiding disturbance from an overly loud playback of the masking sound itself.

Some masking sound generators implement an adaptive function that adjusts the masking sound volume as a function of the background (room) noise volume. This enables automatic volume adjustment of the masking sound based directly on the room noise level.

EP 1 886 707 discloses a device for monitoring noises in a sleeping environment and for generating a calming or soothing sound output to mask the noise and encourage sleep. The device detects ambient noise in a sleeping environment and also monitors the user's vital signs to derive the user's sleep state. Based on the determined sleep state, the device is adapted to control the characteristics of the audio output, such as tempo and volume.

However, a problem with controlling the volume of masking sounds is that the masking sounds themselves may then become a source of disturbance. This may depend on the sensitivity of the user to the masking sounds. There is a desire to optimize the operation of masking sound generators, and in particular to provide suitable masking sounds that minimize or reduce a disturbance to a user.

SUMMARY OF THE INVENTION

The invention is defined by the claims.

According to examples in accordance with an aspect of the invention, there is provided a device for masking noise, the device comprising:

a transducer unit for detecting noise to be masked;
a sound generating unit for generating a masking sound;
a sensor unit for monitoring a user's brain activity; and
a controller adapted to:

during a calibration, determine the user's brain activity, measured by the sensor unit, in response to a calibration sound, generated by the sound generating unit; and

during use of the device, adjust signal characteristics of the masking sound based on:

the noise detected by the transducer unit;
the user's brain activity in response to the noise detected by the transducer unit; and
the user's brain activity in response to the calibration sound generated by the sound generating unit during the calibration.

The device generates a masking sound to mask external noise, e.g. for use during sleep. In examples, the external noise to be masked is one that would otherwise disturb the user's sleep. The device automatically adjusts the signal characteristics of the masking sound based on the noise level in the environment, the user's response to this environmental noise level and the user's sensitivity to the masking sound (as previously determined during a calibration). Thus, the masking sound is able to mask the external noise whilst taking the user's sensitivity to the masking sound into account. This could, for example, mitigate unintentional disturbances to the user (or the user's sleep) due to an overly loud playback of the masking sound itself or disturbances caused by a user's sensitivity to a certain frequency or frequencies of acoustic noise. The sensitivity may be determined during the calibration stage as discussed in more detail below. The method of adjusting the signal characteristics of the masking sound based on the noise detected by the transducer unit is for example particularly suited to masking frequently occurring noises (for example traffic or snoring).

The signal characteristics of the masking sound may comprise at least one of a signal volume and a signal frequency. Thus, the device is able to adjust the loudness, the pitch, the quality, the type, and/or the tone of the masking sound in order to optimally mask the external noise. This function is well suited for noises which occur frequently, recurrently (but unpredictably), with a known frequency, and for a time period longer than a few seconds.

The sound generating unit may be adapted, during the calibration, to generate a calibration sound and the controller is adapted to determine the user's brain activity in response to the calibration sound, set upper and lower volume limits for the masking sound based on the user's brain activity in response to the calibration sound.

These features provide the method of an automatic device calibration that enables the device to set limits for the volume of the masking sound based on the user's hearing sensitivity.

The user's brain activity, measured by the sensor unit, may comprise an electroencephalography (EEG) response. Thus, the sensor unit may comprise an electroencephalography system, e.g. formed of one or more electrodes. Measurement of the EEG response comprises measurement of the auditory steady-state response (ASSR), auditory brainstem response (ABR) or event related potentials (ERPs). These measurements correlate to the sensitivity of the human ear. This information can therefore be used for accurate calibration of the signal characteristics (e.g. volume) of the masking sound.

The device may further comprise a memory storage unit, in communication with the controller, adapted to store noise data based on the noise detected by the transducer unit. The controller may be further adapted to analyze the noise data using an algorithm, determine an expected noise trend, and adjust the signal characteristics of the masking sound based on the expected noise trend. Thus, the device is able to store and analyze the trend of repetitive noises over typical nights, enabling the device to predict the noise level expected to occur in the environment. Predictions can be based on metadata, such as day of the week, time of the year, and previous sleep cycles.

In a further embodiment, the algorithm used to analyze the noise data may be a machine-learning algorithm. The machine-learning algorithm enables the device to improve the prediction of the expected noise trend based on previously recorded data.

The device may further comprise a user-interface unit, in communication with the controller, adapted to enable the user to manually adjust the volume of the masking sound. This enables the user to interact and control features of the device, such as adjusting the volume of the masking sound and performing a manual device calibration.

The sound generating unit may be adapted, during the calibration, to generate a calibration sound, the user-interface unit is adapted to receive user feedback on the calibration sound, and the controller is adapted to set an upper volume limit and a lower volume limit for the masking sound based on the user feedback.

This provides a manual device calibration that enables the user to provide feedback using the user-interface unit, enabling the system to adapt the sound intensity from the sound generating unit to the level of the ASSR, so that differences in audio delivery and reception between nights can be compensated. This calibration can be implemented in conjunction with the automatic device calibration as a supplementary calibration, in order to further improve the accuracy of the device's ability to mask noise.

In a further embodiment, the sensor unit may be further adapted to detect a user's sleep state based on the user's measured brain activity. The controller may be further adapted to adjust the signal characteristics of the masking sound based on the user's sleep state. Thus, the device automatically adjusts the signal characteristics of the masking sound based on the user's sleep state and optionally the timing of transitions between different sleep states.

In a further embodiment, the controller may be further adapted to determine a recommended adjustment of the signal characteristics of the masking sound based on the user's sleep state. The user-interface unit may be further adapted to notify the user of the recommended signal characteristic adjustment. Thus, the device recommends to the user whether the signal characteristic should be adjusted based on the user's sleep state, enabling the user to manually adjust this setting. In particular, the signal characteristics may be adjusted when the system has detected arousals or awakenings (detected based on the determined sleep state).

This procedure may for example involve recommending volume adjustment to the user based on user data relating to the user's sleep pattern collected in previous nights, and in particular relating to detected awakenings, arousals, or regularity of light and deep sleep.

In a preferred embodiment, the sensor unit may be in wireless communication with the controller. This enables the sensor unit to be physically detached from the rest of the device, improving the user comfort should the sensor unit be a wearable component.

The masking sound generated by the sound generating unit may be a continuous calming sound adapted to relax the user. The continuous calming sound improves the user's sleep quality by relaxing the user and reducing stress.

The invention also provides a method for masking a noise, the method comprising:

during a calibration, determining the user's brain activity in response to a calibration sound;
detecting a noise to be masked;
monitoring a user's brain activity; and
generating a masking sound, and adjusting the signal characteristics of the masking sound based on the detected noise, the user's brain activity in response to the noise, and the user's brain activity in response to the calibration sound.

This method provides a masking sound adapted to mask external noise for example for use during sleep. For example the external noise that is masked could otherwise disturb the user's sleep.

The method of calibrating the device may comprise:

generating a calibration sound; and
monitoring the user's brain activity in response to the calibration sound, and

wherein adjusting the signal characteristics of the masking sound comprises:

setting upper and lower volume limits for the masking sound based on the user's brain activity in response to the calibration sound.

This method provides an automatic device calibration that enables setting limits for the volume of the masking sound based on the user's hearing sensitivity. In a further or alternative embodiment, the method of calibrating the device may comprise:

generating a calibration sound;
receiving user feedback on the calibration sound; and
setting an upper volume limit and a lower volume limit for the masking sound based on the feedback provided by the user.

This method provides a manual device calibration. The user may provide feedback on the calibration sound, which enables control of the sound intensity of the calibration sound based on the feedback. This method of calibration may be used as a supplementary calibration method in addition to the automatic calibration method, to further improve the accuracy of the noise masking.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a noise masking device according to an embodiment;
Fig. 2 shows a use-case scenario of the noise masking device in the presence of unwanted acoustic noise;
Fig. 3 shows a first method for calibrating the noise masking device according to an embodiment;
Fig. 4 shows a second method for calibrating the noise masking device according to an embodiment; and
Fig. 5 shows a method for adjusting the signal characteristics of a generated masking sound generated using the noise masking device.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device (and method) for masking noise in which a calibration is carried out to determine the sensitivity of a user to a calibration sound. During use of the device, the signal characteristics of the masking sound are adjusted based on the detected noise, the response of the user to the detected noise and also the response of the user to the calibration sound. As a result, a masking sound is generated that is optimally adapted to mask unwanted noise, in particular in a way which avoids the masking noise itself becoming a disturbance to the particular user.

Embodiments are at least partly based on the realization that, although masking sounds are adapted to mask external noise that may otherwise disturb a user, there exists a risk that the masking sound itself may contribute to the user's disturbance. It has been recognized that it is possible to regulate the masking sound based on the user's sensitivity to the masking sound.

Illustrative embodiments may, for example, be employed to improve a user's sleep quality by reducing the disturbance caused by external noises during a user's sleep.

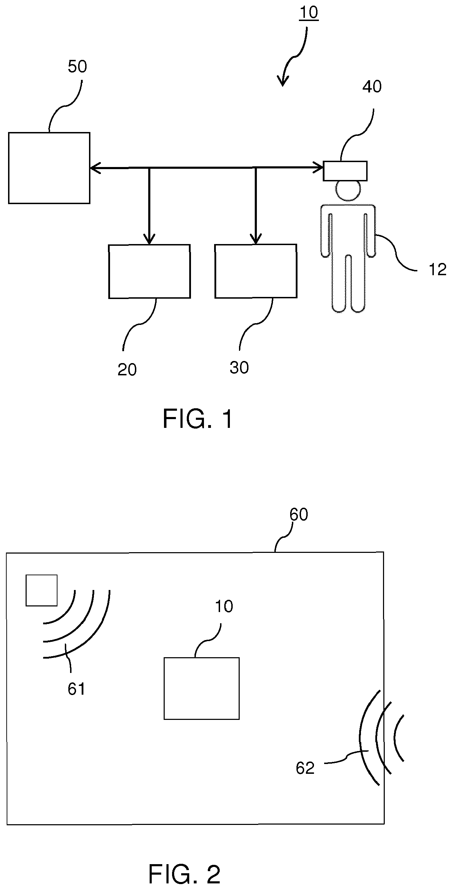

Fig. 1 shows a noise masking device 10, comprising a transducer unit 20 for detecting sound, a sound generating unit 30, a sensor unit 40 for detecting brain activity of the user 12, and a controller 50. The controller 50 is adapted to mask a detected noise using a generated masking sound.

The transducer unit 20 is adapted to detect sound, for example unwanted acoustic noise in an environment of a user 12. The transducer unit 20 may comprise a microphone.

The sound generating unit 30 is adapted to generate sound, for example a continuous tone or repeating tune. The generated sound is adapted to mask noise detected by the transducer unit 20 and can thereby be described as a "masking sound".

The sensor unit 40 is adapted to monitor the user's brain activity. Monitoring of brain activity provides a method of detecting a user's response to a particular sound. In a preferred example, the method of monitoring brain activity may be based on electroencephalography (EEG). Measuring an EEG response may include determining the auditory steady-state response (ASSR) or event related potentials (ERPs) of the user 12. A typical method of measuring an EEG response uses non-invasive electrodes placed along the user's scalp. When a user 12 hears a sound, a measurable EEG response is detected. In a preferred embodiment, the sensor unit 40 may include a wearable headset comprising non-invasive electrodes adapted to measure the user's EEG response.

The controller 50 is in communication with the transducer unit 20, the sound generating unit 30, and the sensor unit 40. In a preferred embodiment, the controller 50 is in wireless communication with at least the sensor unit 40 (and optionally other units as well, such as the sound generating unit). An operation of the controller 50 will be described below.

Fig. 2 shows an example use of the device 10. When in use, the device 10 may be located in an enclosed environment 60, such as a bedroom. Noise detected by the device may include noise 61 with an origin located inside the enclosed environment or noise 62 with an origin located outside the enclosed environment. The detected noise may further comprise a combination of the noise from these origins. Examples of sources of noise 61 from within an enclosed environment include electrical appliances, snoring partners, and insects. Examples of sources of noise 62 from outside an enclosed environment include traffic, aircraft, neighbors, construction work, and insects.

To operate the device, a calibration is first carried out. The purpose of the calibration is to determine the user's brain activity in response to a calibration sound, generated by the sound generating unit 30. This calibration sound is of the same type as will be generated to be a masking sound during subsequent use of the system. Thus, it enables a determination of how the user will respond to the masking sound, and thereby enables control of the masking sound to ensure that the masking sound itself does not become a source of disturbance to the user's sleep.

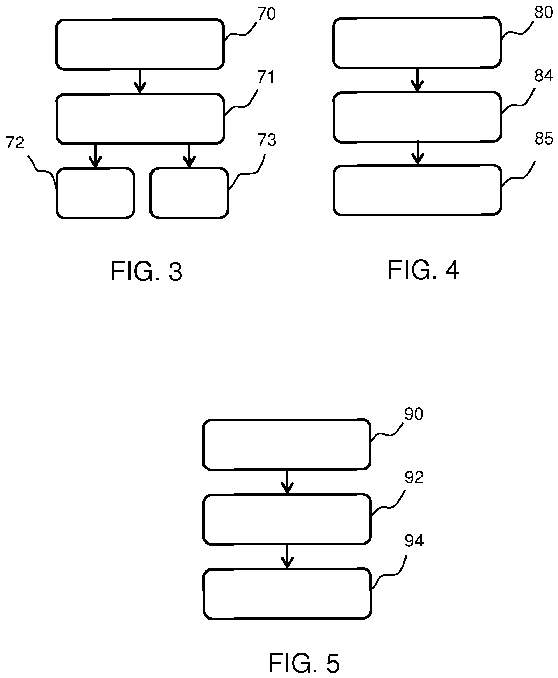

Fig. 3 shows a first possible calibration method, which provides automatic calibration of the device 10. In an initial step 70, the sound generating unit 30 produces a calibration sound. In a step 71, the controller 50 monitors the user's brain activity based on the calibration sound, enabling limits or bounds to be set for the volume of the masking sound. Sounds above the (user specific) threshold will generate a measurable ASSR or ABR, indicating the sensitivity of the user's hearing, i.e. a point at which the user 12 would begin to be disturbed by the volume of the masking sound.

In step 72, the upper limit for the masking sound may therefore be set based on the user's brain activity in response to the calibration sound, whilst in a step 73, the lower limit is determined based the point at which a measurable ASSR or ABR is detected.

For this purpose, the generation of the calibration sound involves sweeping characteristics of the calibration sound, in particular the volume but optionally also the frequency, frequency spectrum or other characteristics, in order to determine the user's response to different sound types. Thus, the characteristics of the user's hearing are determined, such as the volume and/or frequency range of their hearing capabilities.

As explained further below, the result of the calibration may be to derive a calibration constant. This may be set at a default value for the average user, or an average for the user's age group and/or gender. The calibration constant is then adjusted based on a determined ratio of the user's hearing threshold compared to the average hearing threshold. For example, an average hearing threshold for a normal man aged 20 is 3dB at 2kHz. If the user's threshold is determined at 10dB the calibration constant may be set 5 times as high as the default value to achieve a similar perceived noise level for this particular user.

Note that ASSR or ABR detection are only examples. It is also possible to measure the heart rate response to sound, as described in Roessler, R. , Collins, F. and Burch, N. R. (1969), HEART RATE RESPONSE TO SOUND AND LIGHT. Psychophysiology, 5: 359-369.

Fig. 4 shows a second possible calibration method. In step 80, the sound generating unit 30 produces a calibration sound. As discussed above, this involves a sweep of sound characteristics.

In a step 84, the device 10 requests the user's feedback based on the perceived sound level of the masking sound (e.g. low, moderate, high intensity sound) and the comfort level. This may be correlated with the ASSR or ABR response for a personal calibration of the device 10. In a step 85, the upper and lower limits of the masking sound volume may be set based on manual input feedback provided by the user 12 in response to the calibration sound. For example, if the user 12 selects a "low" option, this may correspond to the lower limit, and if the user 12 selects a "high" option, this may correspond to the upper limit. The device may comprise a user-interface unit to enable the user 12 to manually input response information.

The calibrated device 10 is then used to adapt the masking sound volume produced by the sound generating unit 30 to the level of the user's measured ASSR or other measure of hearing characteristics, so that differences in audio delivery and reception due to the user's hearing characteristics but also for changes in positioning of the sound generating unit 30 can be compensated.

Fig. 5 shows a method for masking noise using the device 10 of Fig. 1. This method follows one (or even both) of the calibration methods described above.

In an initial step 90, the transducer unit 20 detects a noise to be masked. In step 92, the sensor unit 40 measures the user's brain activity, which is thus in response to the detected noise.

These two steps enable the noise to be detected by the transducer unit and also enable monitoring of the user's brain activity in response to the noise detected by the transducer unit. Thus, it can be determined if noise masking is needed based on whether the user's brain activity shows that the user has been disturbed.

If noise masking is needed the sound generating unit 30 generates a masking sound in step 94. The masking sound takes account of the previous device calibration. Thus, the signal characteristics of the masking sound are adjusted based on the detected noise, the user's brain activity in response to the noise, and the user's brain activity in response to the calibration sound.

The signal characteristics of the masking sound which are adjusted for example comprise:

the volume;
the frequency (for a single tone);
the frequency spectrum (for a noise based signal), such as a type of noise;
the temporal characteristics such as a volume function over time.

The frequency spectrum may for example be adapted to match the hearing capabilities of the user. For example, a user will have a particular hearing response to different frequencies (as may be determined during the calibration) so that a white noise signal may be adapted so that instead of having a flat amplitude as a function of frequency, the amplitude follows the inverse of the hearing sensitivity of the user, so that the user perceives a white noise sound.

There may be frequencies to which the user is particularly sensitive (either their hearing or their brain response), and these may be suppressed in the masking sound.

Typical examples of noise detected during a period of sleep include traffic noise or snoring sounds. The adjusted signal characteristics typically include the signal volume and/or the signal frequency, although other options are outlined above.

The transducer unit 20 measures the frequency of the detected noise and the sensor unit 40 analyzes the user's EEG spectrum for determination of an ASSR or ABR. The frequency which may enable the detection of an ASSR peak as a result of the detected noise is known by the controller 50.

By way of example, the ASSR is an electrophysiological response to rapid auditory stimuli obtained by applying a carrier stimulus at a certain repetition rate, for instance every 10 milliseconds. Test frequencies used are commonly 500, 1000, 2000, and 4000 Hz. These frequencies are for example amplitude modulated. The brain signal that is recorded in the EEG spectrum is a response to the auditory carrier stimulus and is only present if the auditory carrier stimulus is registered by the ear (i.e. below the hearing threshold no response signal will be present in the EEG spectrum, above the threshold the response is visible in the spectrum).

If the detected noise is registered in the EEG spectrum, the masking sound volume is adjusted to the level of the noise. This function is well suited for sound which occurs frequently and recurrently (but unpredictably), with a known frequency (such as railroad, aircraft, traffic, and snoring sounds) and for a time period longer than a few seconds for each noise-producing event. This enables the masking sound to be optimally tuned to the detected noise, so that the noise masking is improved, with a lower risk of discomfort for the user 12 due to disturbance caused by the masking sound.

The volume of the masking sound is thus adjusted based both on the noise detected by the transducer unit 20 (so that the masking sound is able to mask the noise) and on the user's response (so that the masking sound does not disturb the user).

The user 12 may for example set the volume of the masking sound before going to sleep, such that the masking sound volume is sufficient to mask the unwanted noise at the time the user goes to sleep. If the noise level changes over time the masking sound volume is adjusted accordingly to ensure the unwanted noise remains masked by the masking sound.

The masking sound power set point (i.e. the power of the masking sound at a given time, where power corresponds to sound level) can be defined as:

$$P_{set} = P_{init} + C\left(P_{ext} - P_{ext\_init}\right) \qquad (1)$$

where $P_{set}$ is the masking sound power set point, $P_{init}$ is the initial masking sound power at the volume set by the user 12 at the start of the session, $P_{ext}$ is the sound power of the unwanted noise at a given time, $P_{ext\_init}$ is the initial sound power of the unwanted noise, and C is a calibration constant (e.g. having a default value C=1).

In this way, the masking sound power is initially set to match the unwanted noise, and this may be carried out by the user before they sleep. As the noise changes, the set point is adapted in response to changes in the noise power, taking account of the user's personal characteristics embodied in the calibration constant C.

As mentioned above, the calibration steps may for example involve setting the value of the calibration constant, C as well as setting a maximum for the value $P_{set}$.

The calibration constant may be set automatically or manually based on the user's hearing sensitivity determined during the calibration. With the calibration constant C set at a default level, the system outputs sounds which are audible for an average person. Mappings between calibration constants and hearing characteristics are stored in the system, for instance based on the sound level threshold at which an ASSR is detected. For users with less sensitive hearing, the calibration sequence will detect a larger audio level threshold at which an ASSR response is present and then will set the calibration constant C accordingly, for instance using a Decibel scale.

In a preferred embodiment, the trend of the repetitive noise (e.g. over typical nights) may be stored and analyzed. Based on metadata (such as the day of the week and the time of year) and/or personal data (such as previous sleep/wake times of the user 12), the set point ($P_{set}$) may be further adjusted to better predict the sound level of the detected noise that is expected to occur based on a (typical) reference sound level during such a night ($P_{ext\_ref}$ at time t versus the reference sound level of the unwanted noise at the start of the session (time t0)):

$$P_{set}(t) = P_{init} + C\left(P_{ext} - P_{ext\_init}\right)\left(\frac{P_{ext\_ref}(t)}{P_{ext\_ref}(t0)}\right) \qquad (2)$$

where $P_{ext\_ref}^{(t)}$ is the reference sound power of the unwanted noise at a given time t, and $P_{ext\_ref}^{(t0)}$ is the initial reference sound power at the start of the session.

As shown, the adjustment to the initial power is scaled based on the ratio between the noise level at the particular time and a noise level at a reference time t0.

The value P_ext can is measured at particular set points rather than continuously which means it will be lagging with respect to the external noise level. This is not an issue when the noise decreases over time, but it is a problem when there is an increase in noise during the night. Thus, a predictive element is incorporated which can anticipate the increase in sound ahead of time and ensure that the masking sound is adjusted for it.

This is for example of interest when a large but predictable increase in external noise level occurs during the night, for instance a train passing by or another person's alarm clock.

The noise-masking device may further include a memory storage unit, which stores noise data based on the noise detected by the transducer unit 20. The memory storage unit may be in communication with the controller 50, which may be adapted to analyze the noise data using an algorithm. This algorithm may be a machine-learning algorithm which takes into account previous data to predict the trend of unwanted noise sound level. The controller 50 may be further adapted to determine an expected noise trend based on the noise data analysis. This controller 50 may thereafter adjust the signal characteristics of the masking sound based on the expected noise trend.

The device 10 may include a user-interface unit adapted to enable the user 12 to manually adjust the volume of the masking sound. The user-interface unit may be in communication with the controller 50.

In a certain embodiment, the signal characteristics of the masking sound may be adjusted based on a user's sleep state detected by the sensor unit 40. The sensor unit 40 measures the user's brain activity and derives sleep relevant features such as awakenings, arousals, and regularity of light and deep sleep (e.g. number of exits out of deep sleep).

Based on the sleep relevant features derived from previous sleep sessions, the controller 50 may recommend the user 12 to change the volume or frequency of the masking sound, or automatically adapt the masking sound.

The recommended signal characteristics adjustment may be communicated to the user 12 through a notification displayed using the user-interface unit. In a preferred embodiment, the transducer unit 20 may measure and record data on noise levels during a user's sleeping period in order to produce data on expected noise levels by means of an algorithm. The controller 50 may be adapted to generate the recommended adjustment of the signal characteristics based on the produced data, and the user-interface unit may be adapted to provide the produced data and the ability to adjust the signal characteristics based on the data to the user 12.

The system may additionally be used to generate continuous calming sounds adapted to relax the user 12 when they are going to sleep. This is an additional feature to the masking of noise, and is known.

The calming sound is designed to calm the user 12 in order to ease the process of falling asleep. Thus, in addition to delivering masking sounds during sleep, the system may also be used to play calming sounds to initiate sleep. These calming sounds may also be noise based signals, or they may be other signals, such as speech, whale noises etc.

The sound generating unit 30 may also deliver auditory stimulation while a user 12 is sleeping to enhance sleep slow waves without causing arousals. This is an additional feature to the masking of noise, and is known. Such sounds for example comprise pulses of sound, for example with 50ms duration separated by 1 second pauses. This enables cognitive benefits and enhancement of sleep restoration. There are different causes that can prohibit falling asleep (e. g. before first sleep onset or falling back asleep after waking up later during the night). These are generally categorized in two sets, internal and external causes. External causes may include unwanted acoustic noise, as described above. Internal disturbances may include psychological causes (e.g. stress, rumination), physiological causes (e.g. low sleep pressure, tinnitus, hypertension), and behavioral causes (e.g. poor sleep hygiene). By playing a continuous calming sound during the user's sleep the device 10 may improve the user's sleep quality.

Thus, in addition to delivering masking sounds during sleep, the system may also be used to play sounds to promote deep sleep.

Applications of the invention may include, but are not limited to, any application which includes one or more of a sleep tracking system, a sound delivery system, and a sound level measurement system. For example a cardiorespiratory-based sleep tracker in combination with a smartphone or wake-up light.

The skilled person would be readily capable of developing a controller for carrying out a previously described method. Thus, each step of the flow chart may represent a different action performed by a controller, and may be performed by a respective module of the controller.

As discussed above, embodiments make use of a controller. The controller can be implemented in numerous ways, with software and/or hardware, to perform the various functions required. A processor is one example of a controller which employs one or more microprocessors that may be programmed using software (e.g. microcode) to perform the required functions. A controller may however be implemented with or without employing a processor, and also may be implemented as a combination of dedicated hardware to perform some functions and a processor (e.g. one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

**Claims**

1. A device (10) for masking noise, the device (10) comprising:

    a transducer unit (20) for detecting noise to be masked;
    a sound generating unit (30) for generating a masking sound;
    a sensor unit (40) for monitoring a user's brain activity; and
    a controller (50) adapted to:

        during a calibration, determine the user's brain activity, measured by the sensor unit (40), in response to a calibration sound, generated by the sound generating unit (30); and
        during use of the device (10), adjust signal characteristics of the masking sound based on:

            the noise detected by the transducer unit (20);
            the user's brain activity in response to the noise detected by the transducer unit (20); and
            the user's brain activity in response to the calibration sound generated by the sound generating unit (30) during the calibration.

2. A device (10) as claimed in claim 1, wherein the signal characteristics of the masking sound comprise at least one of:

    a signal volume; and
    a signal frequency.

3. A device as claimed in claim 2, wherein:

    the sound generating unit (30) is adapted, during the calibration, to generate a calibration sound; and
    the controller (50) is adapted to:

        determine the user's brain activity in response to the calibration sound;
        set an upper and lower volume limits for the masking sound based on the user's brain activity in response to the calibration sound.

4. A device (10) as claimed in any of claims 1 to 3, wherein the sensor unit (40) comprises an electroencephalography (EEG) system, so that the user's brain activity measured by the sensor unit (40) comprises an electroencephalography response.

5. A device (10) as claimed in any of claims 1 to 4, further comprising:

    a memory storage unit, in communication with the controller (50), adapted to store noise data based on the noise detected by the transducer unit (20);
    wherein the controller (50) is further adapted to:

        analyze the noise data using an algorithm, preferably wherein the algorithm is a machine-learning algorithm;
        determine an expected noise trend; and
        adjust the signal characteristics of the masking sound based on the expected noise trend.

6. A device as claimed in any of claims 1 to 5, further comprising a user-interface unit, in communication with the controller (50), adapted to enable the user (12) to manually adjust the volume of the masking sound.

7. A device as claimed in claim 6, wherein:

    the sound generating unit (30) is adapted, during the calibration, to generate a calibration sound;
    the user-interface unit is adapted to receive user feedback on the calibration sound; and
    the controller (50) is adapted to set an upper volume limit and a lower volume limit for the masking sound based on the user feedback.

8. A device as claimed in any of claims 1 to 7, wherein:

   the sensor unit (40) is further adapted to detect a user's sleep state; and
   the controller (50) is further adapted to adjust the signal characteristics of the masking sound based on the user's sleep state.

9. A device as claimed in claim 8, wherein:

   the controller (50) is further adapted to determine a recommended adjustment of the signal characteristics of the masking sound based on the user's sleep state; and
   the user-interface unit is further adapted to notify the user (12) of the recommended adjustment.

10. A device as claimed in any of claims 1 to 9, wherein the masking sound generated by the sound generating unit (30) is a continuous calming sound adapted to relax the user (12).

11. A device as claimed in claims 1 to 10, wherein the sensor unit (40) is in wireless communication with the controller (50).

12. A method for masking noise, the method comprising:

    (70,71,72,73; 80,84,85) during a calibration, determining the user's brain activity in response to a calibration sound;
    (90) detecting a noise to be masked;
    (92) monitoring a user's brain activity; and
    (94) generating a masking sound, and adjusting the signal characteristics of the masking sound based on the detected noise, the user's brain activity in response to the noise, and the user's brain activity in response to the calibration sound.

13. A method as claimed in claim 12, wherein the calibration method comprises:

    (70) generating a calibration sound; and
    (71) monitoring the user's brain activity in response to the calibration sound, and

    wherein adjusting the signal characteristics of the masking sound comprises:

    (72,73) setting upper and lower volume limits for the masking sound based on the user's brain activity in response to the calibration sound.

14. A method as claimed in claim 12 or 13, wherein the step of determining the user's brain activity in response to a calibration sound comprises:

    (80) generating a calibration sound;
    (84) receiving user feedback on the calibration sound; and
    (85) setting upper and lower volume limits for the masking sound based on the user feedback.

15. A computer program comprising code means for implementing the method of any one of claims 12 to 14 when said program is run on a computer.

FIG. 1

FIG. 2

70

71

72                    73

FIG. 3

80

84

85

FIG. 4

90

92

94

FIG. 5

Europäisches
Patentamt

European
Patent Office

Office européen
des brevets

## EUROPEAN SEARCH REPORT

Application Number

EP 18 21 2981

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X <br> A | WO 2018/053114 A1 (BOSE CORP [US]) 22 March 2018 (2018-03-22) <br> * abstract; figures * <br> * paragraphs [0006], [0009] - [0010], [0023] - [0031], [0038] - [0051], [0054] * | 1-4,6-15 <br><br> 5 | INV. <br> G10K11/175 <br> A61B5/0476 <br> A61B5/00 <br> A61M21/00 |
| A | WO 2018/166625 A1 (ERICSSON TELEFON AB L M [SE]) 20 September 2018 (2018-09-20) <br> * abstract; figures * <br> * paragraphs [0001], [0008] - [0013], [0035] - [0049], [0058] - [0063] * | 1-15 | |
| A,D | EP 1 886 707 A1 (FUTURE ACOUSTIC LLP [GB]) 13 February 2008 (2008-02-13) <br> * the whole document * | 1-15 | |

TECHNICAL FIELDS SEARCHED (IPC)

A61B
A61M
H04R
G10K

The present search report has been drawn up for all claims

| Place of search | Date of completion of the search | Examiner |
|---|---|---|
| The Hague | 20 May 2019 | Scappazzoni, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 671 729 A1

## ANNEX TO THE EUROPEAN SEARCH REPORT
## ON EUROPEAN PATENT APPLICATION NO.

EP 18 21 2981

This annex lists the patent family members relating to the patent documents cited in the above-mentioned European search report.
The members are as contained in the European Patent Office EDP file on
The European Patent Office is in no way liable for these particulars which are merely given for the purpose of information.

20-05-2019

| Patent document cited in search report | | Publication date | Patent family member(s) | | Publication date |
|---|---|---|---|---|---|
| WO 2018053114 | A1 | 22-03-2018 | US WO | 2018078732 A1 2018053114 A1 | 22-03-2018 22-03-2018 |
| WO 2018166625 | A1 | 20-09-2018 | NONE | | |
| EP 1886707 | A1 | 13-02-2008 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1886707 A

**Non-patent literature cited in the description**

- **ROESSLER, R. ; COLLINS, F. ; BURCH, N. R.** HEART RATE RESPONSE TO SOUND AND LIGHT. *Psychophysiology,* 1969, vol. 5, 359-369